## Europäisches Patentamt

## European Patent Office

## Office européen des brevets

(11) Veröffentlichungsnummer: **0 037 920**
**A1**

## (12) EUROPÄISCHE PATENTANMELDUNG

(21) Anmeldenummer: **81102100.5**

(22) Anmeldetag: **20.03.81**

(51) Int. Cl.³: **A 61 M 5/315**

(30) Priorität: **10.04.80 DE 8009848 U**

(43) Veröffentlichungstag der Anmeldung: **21.10.81**
**Patentblatt 81/42**

(84) Benannte Vertragsstaaten: **AT BE CH DE FR GB IT LI LU NL SE**

(71) Anmelder: **Intermedicat GmbH, Gerliswilstrasse 45, CH-6020 Emmenbrücke (CH)**

(72) Erfinder: **Lesemann, Egon, Empfershausen Berliner Strasse, D-3501 Körle (DE)**

(74) Vertreter: **von Kreisler, Alek et al, Deichmannhaus am Hauptbahnhof, D-5000 Köln 1 (DE)**

(54) **Injektionsspritze.**

(57) Bei einer Injektionsspritze (1) ist es wichtig, daß die aufgezogene Menge der Arzneimittellösung vollständig wieder ausgeschoben und appliziert wird, damit kein Arzneimittelverlust durch Restvolumina in der Injektionsspritze (1) auftritt. Zu diesem Zweck ist vorgesehen, daß der Kolben (5) einen axial gerichteten Verdrängerstift (8) aufweist, der in den rohrförmigen Anschlußstutzen (7) des Spritzengehäuses eintaucht und diesen unter Beibehaltung eines axialen Kanals (14) im wesentlichen ausfüllt. Beim Vorschub des Kolbens (5) wird der Verdrängerstift (8) in das Innere des rohrförmigen Anschlußstutzens (7) eingefahren, so daß das darin befindliche Restvolumen bis auf einen Benetzungsfilm durch den verengten axialen Kanal (14) im Anschlußstutzen (7) ausgeschoben wird. Das Restvolumen wird auf diese Weise auf fast Null reduziert.

**VON KREISLER   SCHÖNWALD   EISHOLD   FUES**
**VON KREISLER   KELLER   SELTING   WERNER**

PATENTANWÄLTE

Dr.-Ing. von Kreisler † 1973
Dr.-Ing. K. Schönwald, Köln
Dr.-Ing. K. W. Eishold, Bad Soden
Dr. J. F. Fues, Köln
Dipl.-Chem. Alek von Kreisler, Köln
Dipl.-Chem. Carola Keller, Köln
Dipl.-Ing. G. Selting, Köln
Dr. H.-K. Werner, Köln

Sg-DB/my   U9. April 1980

DEICHMANNHAUS AM HAUPTBAHNHOF
**D-5000 KÖLN 1**

0037920

B. BRAUN MELSUNGEN AG
Carl-Braun-STraße

3508 Melsungen

Injektionsspritze

Die Erfindung betrifft eine Injektionsspritze mit einem im wesentlichen zylindrischen Spritzengehäuse, in dem ein Kolben mittels einer Kolbenstange verschiebbar ist und an dessen einem Ende ein rohrförmiger Anschlußstutzen zum Anschluß einer Injektionsnadel, einer Kanüle o.dgl. angeordnet ist.

Injektionsspritzen der in Rede stehenden Art sind in verschiedenen Formen aus der Praxis bekannt. Derartige Injektionsspritzen werden auf vielen Gebieten der Technik, insbesondere aber auf dem Gebiet der Medizin verwendet, dort insbesondere zur Applikation von Arzneimittellösungen. Als Materialien zur Herstellung von Injektionsspritzen sind Glas, Kunststoff, für Kolben auch elastische Materialien, wie Gummi, für äußere Schutzhüllen auch Metall, sowie Kombinationen dieser Materialien bekannt.

Injektionsspritzen werden, unabhängig davon, ob sie mehrfach verwendbar oder nur einmal verwendbar sind, entweder mit feststehenden, untrennbar mit dem Spritzengehäuse verbundenen Injektionsnadeln oder Kanülen, oder aber mit auswechselbaren Injektionsnadeln oder Kanülen hergestellt. Vor allem im letztgenannten Fall weist das Spritzengehäuse zumeist einen rohrförmigen Anschlußstutzen zum Anschluß einer Injektionsnadel, einer Kanüle o.dgl. auf. Es hat sich nun in der Praxis gezeigt, daß bei allen bislang bekannten Injektionsspritzen mit derartigen rohrförmigen Anschlußstutzen die aufgezogene Menge der Arzneimittellösung nicht vollständig wieder ausgeschoben und damit appliziert werden kann, da durch den Vorschub des Kolbens die Arzneimittellösung nur aus dem Inneren des Spritzengehäuses, nicht aber aus dem Inneren des Anschlußstutzens ausgeschoben werden kann. Insbesondere bei teuren Arzneimitteln gehen in Form dieses Restvolumens erhebliche Werte einfach verloren.

Ausgehend von dem zuvor erläuterten Stand der Technik liegt der Erfindung die Aufgabe zugrunde, die bekannte Injektionsspritze so auszugestalten und weiterzubilden, daß das nicht ausschiebbare Restvolumen auf ein Minimum reduziert ist.

Diese Aufgabe wird dadurch gelöst, daß der Kolben einen axial gerichteten Verdrängerstift aufweist, der in den rohrförmigen Anschlußstutzen eintaucht und diesen unter Beibehaltung eines axialen Kanals im wesentlichen ausfüllt.

Beim Vorschub des Kolbens wird der Verdrängerstift in das Innere des rohrförmigen Anschlußstutzens eingefahren, so daß das darin befindliche Restvolumen bis auf einen Benetzungsfilm durch den verengten axialen Kanal im Anschlußstutzen ausgeschoben wird. Das Restvolumen wird auf diese Weise auf fast Null reduziert.

Ferner ist vorteilhaft, daß bekannte Injektionsspritzen ohne konstruktive Änderungen des Spritzengehäuses auf die Erfindung umgerüstet werden können, da die Graduierung (Volumeneichung) der Injektionsspritze durch die erfindungsgemäße Konstruktion nicht geändert wird. Das insgesamt aufziehbare Volumen ist nämlich bei der erfindungsgemäßen Konstruktion gerade um das nunmehr ausschiebbare Restvolumen vermindert.

Hinsichtlich der Auswahl der Materialien macht die Erfindung an sich keinerlei Änderungen notwendig. Es können also weiterhin Glas, Kunststoff, elastische Materialien, wie Gummi usw. verwendet werden. Insbesondere dann aber, wenn ein Kolben aus Kunststoff Verwendung findet, ist es besonders vorteilhaft, den Kolben und den Verdrängerstift einstückig miteinander auszubilden. Kolben, ggf. Kolbenstange und Verdrängerstift, können in diesem Fall ohne Schwierigkeit als ein einziges Spritzteil hergestellt werden.

Der bei Eintauchen des Verdrängerstiftes zum Ausstoß der Restmenge erforderliche axiale Kanal kann vorteilhaft dadurch gebildet werden, daß der Durchmesser des Querschnittes des Verdrängerstiftes etwas kleiner ist als derjenige des Durchganges des Anschlußstutzens. Ferner ist es möglich, daß bei zylindrischem Durchgang des Anschlußstutzens der Querschnitt des Verdrängerstiftes mehreckig gestaltet ist.

Auch kann der zylindrische Durchgang des Anschlußstutzens und/oder der Verdrängerstift mindestens eine axial verlaufende Rille aufweisen, die sich über ihre ganze Länge erstreckt. In weiterer vorteilhafter Ausgestaltung der Erfindung weist der zylindrische Durchgang des Anschlußstutzens oder der Verdrängerstift axial verlaufende Rippen auf, die sich über ihre ganze Länge erstrecken.

Die Länge des Verdrängerstiftes entspricht im wesentlichen der Länge des Anschlußstutzens.

Nicht immer sind Injektionsspritzen der in Rede stehenden Art rotationssymmetrisch aufgebaut, insbesondere ist es häufig so, daß der rohrförmige Anschlußstutzen nicht koaxial zu dem Spritzengehäuse angeordnet ist. In diesem Fall muß natürlich auch der Verdrängerstift außermittig angeordnet sein. In diesem Fall ist es notwendig, daß das Spritzengehäuse mit einer Drehsicherung für den Kolben und/oder die Kolbenstange versehen ist, da nur dann gewährleistet ist, daß der Verdrängerstift stets in den rohrförmigen Anschlußstutzen eingeschoben werden kann. Die Drehsicherung kann bei einer Spritze mit kreuzförmiger Kolbenstange so verwirklicht sein, daß das Spritzengehäuse einerseits und der Kolben bzw. die Kolbenstange andererseits mit zueinander komplementären Ausformungen versehen sind. Konstruktiv einfacher ist aber, daß an der Griffplatte des Spritzengehäuses ein Aufsteckschuh befestigt ist, der eine Schlitzausnehmung zum drehsicheren Durchlaß eines Schenkels des Kreuzprofils der Kolbenstange aufweist. Ist die Kolbenstange anders als kreuzförmig gestaltet, so ist die Drehsicherung mit entsprechend angepaßter Ausnehmung zu versehen.

0037920

Es ist bei Injektionsspritzen der in Rede stehenden Art bekannt, den Kolben auf seiner der Injektionsmittelöffnung des Spritzengehäuses zugewandten Seite mit einem Dichtungsteil aus elastischem Material zu versehen, so daß die eigentliche Kolbenwirkung über dieses Dichtungsteil erzielt wird. In diesem Fall kann der an den Kolben angespritzte Verdrängerstift durch eine Öffnung in dem Dichtungsteil nach vorne hindurchragen. Dies empfiehlt sich insbesondere dann, wenn der Kolben und der Verdrängerstift einstückig miteinander ausgebildet sind. Alternativ ist es bei Vorliegen eines Dichtungsteiles aus elastischem Material aber auch möglich, den Verdrängerstift als Fortsatz einstückig an das Dichtungsteil anzuformen.

Im folgenden wird die Erfindung anhand einer lediglich Ausführungsbeispiele darstellenden Zeichnung näher erläutert.

Es zeigen

Fig. 1 im Längsschnitt ein erstes Ausführungsbeispiel einer erfindungsgemäßen Injektionsspritze mit zurückgezogenem Kolben,

Fig. 2 den Gegenstand nach Figur 1 mit vorgeschobenem Kolben,

Fig. 3 im Längsschnitt ein zweites Ausführungsbeispiel einer erfindungsgemäßen Injektionsspritze mit zurückgezogenem Kolben,

Fig. 4 den Gegenstand nach Figur 3 mit voll vorgeschobenem Kolben,

Fig. 5 im Längsschnitt ein drittes Ausführungsbeispiel einer erfindungsgemäßen Injektionsspritze mit zurückgezogenem Kolben,

Fig. 6 im Längsschnitt ein viertes Ausführungsbeispiel einer erfindungsgemäßen Injektionsspritze, und

Fig. 7 einen Schnitt durch den Gegenstand nach Figur 6 entlang der Linie VII-VII.

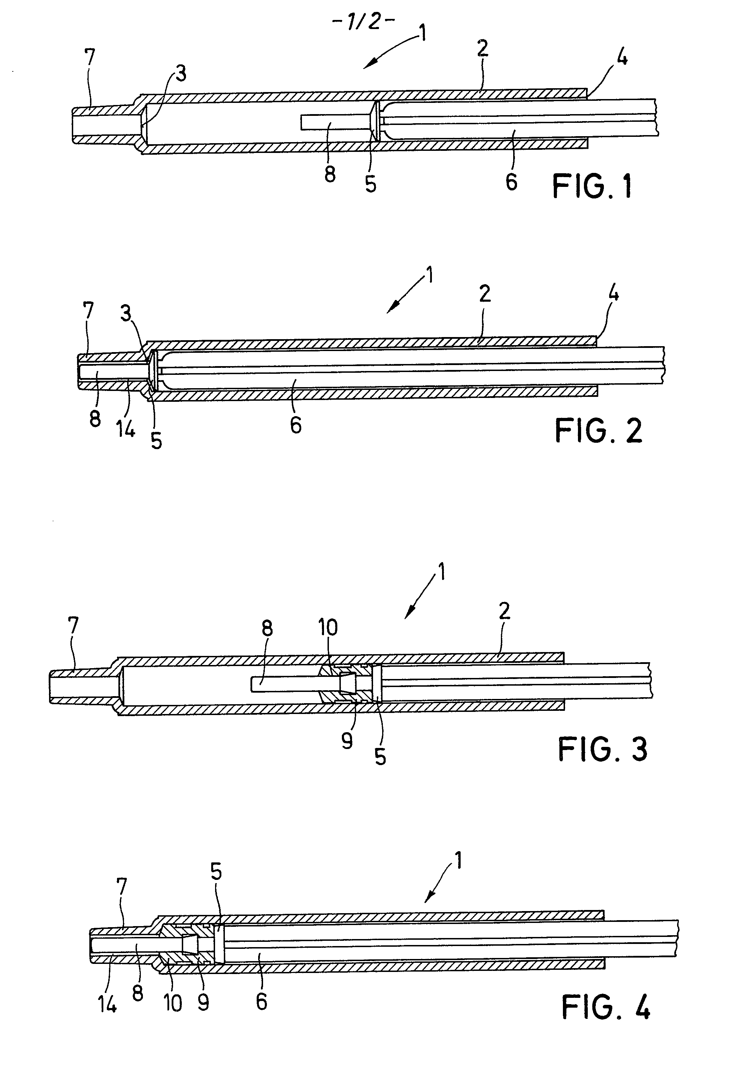

Die in den Figuren 1 bis 7 in verschiedenen Ausführungsbeispielen dargestellte Injektionsspritze 1 weist zunächst ein hohles, im wesentlichen zylinderförmiges Spritzengehäuse 2 mit einer Injektionsmittelöffnung 3 an einer Stirnseite und einer Betätigungsöffnung 4 an der anderen Stirnseite, einen in dem Spritzengehäuse 2 laufenden Kolben 5 und eine auf der der Betätigungsöffnung 4 des Spritzengehäuses 2 zugewandten Seite des Kolbens 5 angeschlossene Kolbenstange 6 zur Betätigung des Kolbens 5 auf. Das Spritzengehäuse 2 weist dabei an der Injektionsmittelöffnung 3 einen rohrförmigen Anschlußstutzen 7 zum Anschluß einer (nicht dargestellten) Injektionsnadel, Kanüle o.dgl. auf.

Aus den Figuren 1 bis 6 ergibt sich weiterhin, daß der Kolben 5 der Injektionsspritze 1 auf seiner der Injektionsmittelöffnung 3 des Spritzengehäuses 2 zugewandten Seite mit einem Verdrängerstift 8 versehen ist, dessen Länge und Anordnung im wesentlichen der Länge und der Anordnung des rohrförmigen Anschlußstutzens 7 angepaßt sind. Bei in den Durchgang des Anschlußstutzens 7 eingeschobenem Verdrängerstift 8 verbleibt zwischen beiden ein Ringkanal 14, durch den das Restvolumen ausgestoßen wird. Der Ringkanal 14 ergibt sich dadurch, daß der Durchmesser des Querschnittes des Verdrängerstiftes 8 etwas kleiner ist als derjenige des Durchganges des Anschlußstutzens 7.

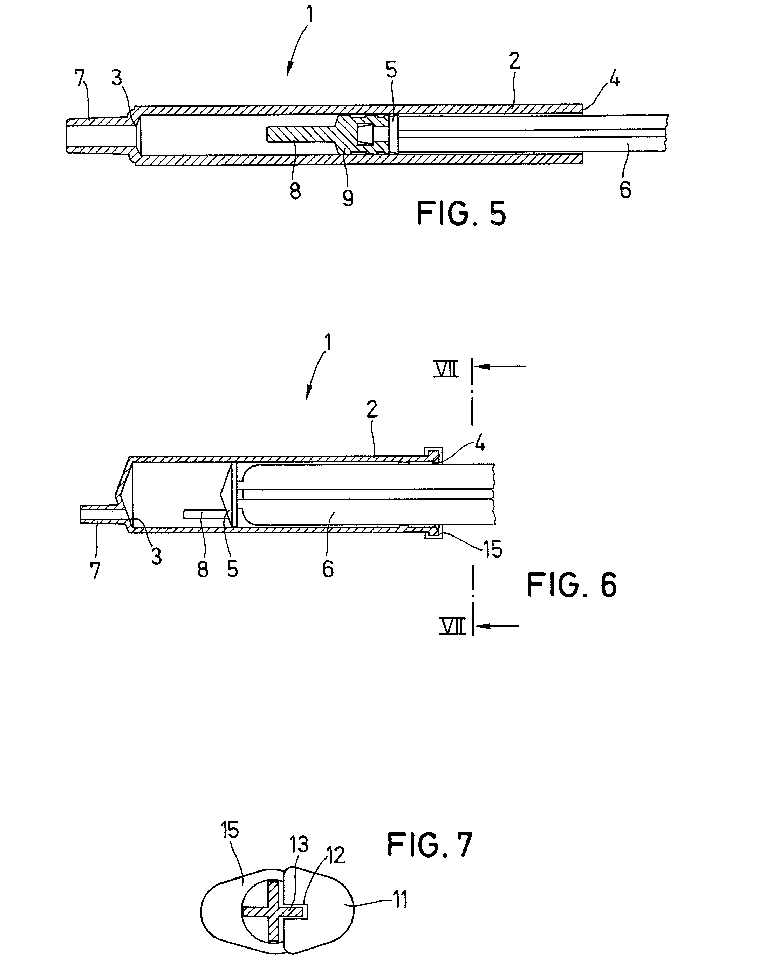

Bei dem in den Figuren 1 und 2 dargestellten Ausführungsbeispiel einer Injektionsspritze 1 sind der Kolben 5, die Kolbenstange 6 und der Verdrängerstift 8 einstückig miteinander ausgebildet. In den Ausführungsbeispielen nach den Figuren 3,4 und 5 ist der Kolben 5 der Injektionsspritze 1 auf seiner der Injektionsmittelöffnung 3 des Spritzengehäuses 2 zugewandten Seite mit einem Dichtungsteil 9 aus elastischem Material versehen. Im Ausführungsbeispiel nach den Figuren 3 und 4 ragt dabei der Verdrängerstift 8 durch eine Öffnung 10 in dem Dichtungsteil 9 hindurch, während im Ausführungsbeispiel nach Figur 5 das Dichtungsteil 9 und der stiftförmige Ansatz 8 einstückig miteinander ausgebildet sind.

Das Ausführungsbeispiel nach Figur 6 unterscheidet sich von den Ausführungsbeispielen der Figuren 1 bis 5 dadurch , daß der rohrförmige Anschlußstutzen 7 des Spritzengehäuses 2 der Injektionsspritze 1 nicht koaxial zu dem Spritzengehäuse 2, sondern außermittig angeordnet ist. Dementsprechend ist auch der Verdrängerstift 8 an dem Kolben 5 außermittig, und zwar koaxial zu dem rohrförmigen Anschlußstutzen 7, angeordnet. Wie sich aus Figur 7 ergibt, ist bei diesem Ausführungsbeispiel einer Injektionsspritze 1 das Spritzengehäuse 2 mit einer Drehsicherung für die kreuzförmige Kolbenstange 6 versehen, so daß die Ausrichtung des Verdrängerstiftes 8 gegenüber dem rohrförmigen Anschlußstutzen 7 stets erhalten bleibt. Die Drehsicherung besteht aus einem Aufsteckschuh 11, der an der Griffplatte 15 an der Betätigungsöffnung 4 des Spritzengehäuses 2 befestigt ist. Der Aufsteckschuh weist eine Schlitzausnehmung 12 auf, in der ein Schenkel 13 der im Querschnitt kreuzförmigen Kolbenstange 6 gleitet.

ANSPRÜCHE

1. Injektionsspritze mit einem im wesentlichen zylindrischen Spritzengehäuse, in dem ein Kolben mittels einer Kolbenstange verschiebbar ist und an dessen einem Ende ein rohrförmiger Anschlußstutzen zum Anschluß einer Injektionsnadel, einer Kanüle o.dgl. angeordnet ist, d a d u r c h   g e k e n n z e i c h n e t,   daß der Kolben (5) einen axial gerichteten Verdrängerstift (8) aufweist, der in den rohrförmigen Anschlußstutzen (7) eintaucht und diesen unter Beibehaltung eines axialen Kanals (14) im wesentlichen ausfüllt.

2. Injektionsspritze nach Anspruch 1,   d a d u r c h   g e k e n n z e i c h n e t, daß der Durchmesser des Querschnittes des Verdrängerstiftes (8) etwas kleiner ist als derjenige des Durchganges des Anschlußstutzens (7).

3. Injektionsspritze nach Anspruch 1,   d a d u r c h   g e k e n n z e i c h n e t,   daß bei zylindrischem Durchgang des Anschlußstutzens (5) der Querschnitt des Verdrängerstiftes (8) mehreckig gestaltet ist.

4. Injektionsspritze nach Anspruch 1,   d a d u r c h   g e k e n n z e i c h n e t,   daß der zylindrische Durchgang des Anschlußstutzens (7) und/oder der Verdrängerstift (8) mindestens eine axial verlaufende Rille aufweist, die sich über ihre ganze Länge erstreckt.

5. Injektionsspritze nach Anspruch 1, d a d u r c h
g e k e n n z e i c h n e t, daß der zylindrische
Durchgang des Anschlußstutzens (7) oder der Verdrängerstift (8) axial verlaufende Rippen aufweist, die
sich über ihre ganze Länge erstrecken.

6. Injektionsspritze nach Anspruch 1, d a d u r c h
g e k e n n z e i c h n e t, daß in dem Verdrängerstift
eine Axialbohrung ausgebildet ist, die auf seinem Umfang
mündet.

7. Injektionsspritze nach einem den Ansprüchen 1 bis 6,
d a d u r c h g e k e n n z e i c h n e t, daß
die Länge des Verdrängerstiftes (8) der Länge des Anschlußstutzens (7) im wesentlichen entspricht.

8. Injektionsspritze nach den Ansprüchen 1 bis 7 mit
außermittig angeordnetem Anschlußstutzen und einer kreuzförmigen Kolbenstange, d a d u r c h g e k e n n -
z e i c h n e t, daß an der Griffplatte (15) des
Spritzengehäuses (2) ein Aufsteckschuh (11) befestigt
ist, der eine Schlitzausnehmung (12) zum drehsicheren
Durchlaß eines Schenkels (13) des Kreuzprofiles der
Kolbenstange (6) aufweist.

9. Injektionsspritze nach den Ansprüchen 1 bis 9,
d a d u r c h g e k e n n z e i c h n e t, daß
der Kolben (5) und der Verdrängerstift (8) einstückig
miteinander ausgebildet sind.

10. Injektionsspritze nach den Ansprüchen 1 bis 9, wobei der Kolben mit einem Dichtungsteil aus elastischem Material versehen ist, d a d u r c h   g e k e n n - z e i c h n e t,   daß der an den Kolben (5) angespritzte Verdrängerstift (8) durch eine Öffnung (10) in dem Dichtungsteil (9) hindurchragt.

11. Injektionsspritze nach den Ansprüchen 1 bis 9, wobei der Kolben mit einem Dichtungsteil aus elastischem Material versehen ist, d a d u r c h   g e k e n n z e i c h - n e t,   daß der Verdrängerstift (8) als Fortsatz einstückig an das Dichtungsteil (9) angeformt ist.

FIG. 1

FIG. 2

FIG. 3

FIG. 4

0037920

FIG. 5

FIG. 6

FIG. 7

Europäisches
Patentamt

EUROPÄISCHER RECHERCHENBERICHT

0037920
Nummer der Anmeldung

EP 81 10 2100.5

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE − C − 883 328 (FESTER) <br> * Fig. 1 * | 1,2,7 |
| | −− | |
| | FR − A − 419 032 (STE ELECTRO-INDUSTRI-ELLE ET ANCIENS ETABLISSEMENTS MATHIEU REUNIS) <br> * Fig. 1 * | 1,2,7 |
| | −− | |
| | FR − A − 1 242 553 (DANIELSSON et al.) <br> * Fig. 13 * | 1,2,7 |
| | −− | |
| | FR − A − 2 224 174 (AMERICAN HOSPITAL SUPPLY CORP.) <br> * Fig. 3 * | 1,2,9 |
| | −−− | |
| | FR − A1 − 2 380 785 (KOMMANDIITTIYHTIO FINNPIPETTE OSMO A. SUOVANIEMI) <br> * Fig. 2 * <br> & DE − A − 2 804 788 | 1,2,9 |
| | −− | |
| | GB − A − 770 341 (DUNMIRE) <br> * Fig. 9 * | 1,2 |
| | −−− | |
| | AU − B − 445 288 (AMERICAN HOSPITAL SUPPLY CORP.) <br> * Fig. 2 * | 1,2,9 |
| | −−−− | |

**KLASSIFIKATION DER ANMELDUNG (Int. Cl.³)**

A 61 M  5/315

**RECHERCHIERTE SACHGEBIETE (Int. Cl.³)**

A 61 M  5/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X | Der vorliegende Recherchenbericht wurde für alle Patentansprüche erstellt.

| Recherchenort | Abschlußdatum der Recherche | Prüfer |
|---|---|---|
| Berlin | 02-07-1981 | ZAPP |

EPA form 1503.1  06.78